# EUROPÄISCHE PATENTSCHRIFT

(11) **EP 2 947 756 B1**
(45) Veröffentlichungstag und Bekanntmachung des Hinweises auf die Patenterteilung: **19.07.2017**
(21) Anmeldenummer: 15168992.4
(22) Anmeldetag: 22.05.2015
(51) Int. Cl.: H02K 11/215, H02K 41/035

(54) **POSITIONSGEREGELTER ELEKTRODYNAMISCHER LINEARANTRIEB**
POSITION CONTROLLED ELECTRODYNAMIC LINEAR MOTOR
ENTRAÎNEMENT LINÉAIRE ÉLECTRODYNAMIQUE À POSITION RÉGLABLE

(30) Priorität: 23.05.2014 DE 102014107297
(43) Veröffentlichungstag der Anmeldung: 25.11.2015
(73) Patentinhaber: Karl Storz GmbH & Co. KG, 78532 Tuttlingen (DE)
(72) Erfinder: Kelp, Martin, 12205 Berlin (DE)
(74) Vertreter: Witte, Weller & Partner Patentanwälte mbB

(56) Entgegenhaltungen:
- DE-A1- 2 946 147
- DE-A1- 4 012 832
- DE-A1-102006 040 779
- DE-A1-102008 038 926
- JP-A- 2000 092 809
- US-A1- 2004 021 458
- US-A1- 2012 098 469

## Beschreibung

### Technisches Gebiet

Die Erfindung betrifft einen Linearmotor insbesondere für optische Systeme. Derartige optische Systeme werden beispielsweise in Endoskopen eingesetzt. Bei modernen Videoendoskopen sind ein Kamerachip sowie ein zugehöriges Linsensystem in die Endoskopspitze integriert. Zur Einstellung der Brennweite bzw. des Fokus des Linsensystems wird ein miniaturisierter Motor benötigt.

### Stand der Technik

Klassische Endoskope, wie sie beispielsweise für die minimalinvasive Chirurgie eingesetzt werden können, führen das Bild mittels Stablinsen vom intrakorporalen Objektiv zum extrakorporalen Okular. Durch die Stablinsen ist das System starr und in der optischen Qualität begrenzt. Moderne Videoendoskope verwenden einen Kamerachip in der Endoskopspitze. Ein solches Endoskop ist in der US 7,365,768 B1 offenbart. Dieses hat vor dem Kamerachip eine starr angeordnete Linse. Eine Einstellung der Brennweite der Linse ist nicht möglich.

In der DE 103 23 629 A1 ist ein Wanderfeld-Linearmotor offenbart, der mindestens drei Statorspulen aufweist. Durch eine phasenverschobene Bestromung der Spulen wird ein Wanderfeld erzeugt, welches eine Verschiebung des Läufers mit axialen Permanentmagneten bewirkt.

Aus der DE 10 2008 038 926 A1 ist ein Linearantrieb bekannt, der zwei axial polarisierte Permanentmagnete im Läufer aufweist. Der Läufer wird durch die Bestromung der Statorspulen in axialer Richtung ausgelenkt. Zusätzlich werden die stabilen Positionen des Läufers durch die im Stator angebrachten Polschuhe realisiert, so dass eine kontinuierliche Verschiebung des Läufers in einem Hüllrohr ermöglicht wird.

In der DE 10 2010 000 582 A1 ist ein weiterer Linearantrieb offenbart, der einen axial polarisierten Permanentmagneten im Läufer und einen oder zwei axial polarisierte Permanentmagnete im Stator aufweist.

Diese drei Linearantriebe umfassen einen Stator und einen Läufer. Die Läufer sind jeweils aus einem oder mehreren Permanentmagneten aufgebaut. Zur Umlenkung und zum Erzeugen des magnetischen Flusses in definierte Richtungen befinden sich an den Permanentmagneten Ringe aus Weicheisen (Polschuhe). Im Stator erzeugen eine oder mehrere Spulen Lorentzkräfte. Teilweise dienen zusätzliche Permanentmagnete und Ringe aus Weicheisen der Erzeugung von Reluktanzkräften. Umschlossen wird der Stator durch eine weichmagnetische Hülse, die das Rückflussjoch für den magnetischen Fluss darstellt. Im stromlosen Zustand befindet sich der Läufer aufgrund von rücktreibenden Reluktanzkräften in einer so genannten Ruheposition. Durch die Speisung der Spulen mit einem elektrischen Strom fester Stromstärke entstehen Lorentzkräfte, die zu einer kontinuierlichen Auslenkung des Läufers aus der Ruheposition führen. Durch das Einstellen eines Kräftegleichgewichts aus Lorentzkräften in der DE 103 23 629 A1 oder aus Lorentz- und Reluktanzkraft in der DE 10 2008 038 926 A1 bzw. der DE 10 2010 000 582 A1 verharrt der Läufer auf dieser ausgelenkten Position. Dabei ist man bestrebt, die Größe der Ströme festen Positionen des Läufers zuzuordnen. Dies wird in der Regel durch Kalibrierung nach dem Aufbau der Antriebe erreicht.

Von außen einwirkende, schwer kontrollierbare Widerstandskräfte, wie beispielsweise die Reibungskraft oder die Schwerkraft, führen zur Positionierungenauigkeit. Durch die Vorgabe fester Stromstärken lässt sich demnach die Position des Läufers nur bedingt festlegen.

Die DE 196 05 413 A1 offenbart einen Linearantrieb mit Wegmessung. Es wird hier die Antriebswicklung gleichzeitig als Messwicklung benutzt. Durch eine solche Wegmessung lässt sich eine höhere Positioniergenauigkeit erreichen. Allerdings ist die Genauigkeit des Mess- und damit des Regelungssystems durch die geringe Änderung der Spuleninduktivität bei der Bewegung des Läufers begrenzt.

In der US 5,747,952 ist ein dreiphasiger Linearantrieb offenbart, bei dem durch einen Hall-Sensor zwischen Spule und Läufer das Magnetfeld gemessen und die Amplitude des Steuersignals auf einen konstanten Wert geregelt wird.

### Darstellung der Erfindung

Der Erfindung liegt die Aufgabe zugrunde, einen Linearmotor dahingehend zu verbessern, dass der Läufer in definierte Positionen gebracht werden kann bzw. eine definierte und vorzugsweise eine lineare Kennlinie zwischen einem Steuersignal und einer Läuferposition besteht. Weiterhin soll bevorzugt die Position des Läufers in weiten Grenzen unabhängig von einer äußeren Last sein. Der Linearmotor soll soweit miniaturisierbar sein, dass er in ein Endoskop integrierbar ist.

Diese Aufgabe wird durch eine Vorrichtung nach einem der unabhängigen Ansprüche gelöst. Vorteilhafte Ausgestaltungen der Erfindung sind in den Unteransprüchen angegeben.

Der erfindungsgemäße Linearmotor umfasst einen Stator sowie einen dazu linear verschiebbaren Läufer. Bevorzugt ist der Linearmotor weitgehend rotationssymmetrisch aufgebaut. Dabei sind die Komponenten weitgehend ringförmig ausgeführt. Die Bewegung des Läufers erfolgt bevorzugt entlang einer Achse, welche bevorzugt parallel zur Mittelachse der rotationssymmetrischen Anordnung liegt und besonders bevorzugt mit dieser identisch ist. Der Stator hat eine oder zwei Spulen, wobei bevorzugt in axialer Richtung seitlich zu den Spulen wenigstens ein Statorpolschuh, sowie in radialer Richtung an der Außenseite ein Rückschlußelement, welches zumindest weitgehend parallel zur Bewegungsrichtung angeordnet ist und bevorzugt die eine oder zwei Spulen und besonders bevorzugt wenigstens einen Statorpolschuh umschließt. Der Läufer wird in radialer Richtung zumindest teilweise von der Spule umschlossen und hat einen ersten Permanentmagneten sowie optional einen zweiten Permanentmagneten. Bevorzugt ist seitlich in axialer Richtung an den Permanentmagneten jeweils ein Läuferpolschuh angeordnet. Es ist im Falle von zwei Permanentmagneten zwischen den beiden Permanentmagneten bevorzugt noch ein Polschuh angeordnet. Die Läuferpolschuhe ermöglichen einen definierten Austritt der Magnetfelder der Permanentmagnete durch die Spule in Richtung des Rückschlußelements. Grundsätzlich arbeitet die Anordnung auch ohne Polschuhe. Durch die Polschuhe kann jedoch die Kraft des Motors um mehr als eine Größenordnung erhöht werden. Im Falle von zwei Permanentmagneten, sind diese axial magnetisiert und mit ihrer Polung so ausgerichtet, dass sich wahlweise die Nordpole oder die Südpole gegenüber liegen. Der Läufer und insbesondere die Permanentmagnete sowie die Polschuhe sind bevorzugt hohlzylindrisch, d.h. sie haben die Form einer zylindrischen Hülse. Der Strahlengang eines optischen Systems kann dann durch die Hülse verlaufen. Insbesondere kann eine Linse oder ein anderes optisches Element in der Hülse sitzen. Somit kann durch eine Verschiebung der Hülse die Brennweite und/oder auch der Fokus des optischen Systems eingestellt werden.

Die Polschuhe und das Rückschlußelement umfassen bevorzugt ferromagnetische und/oder weichmagnetische Materialien.

Der Linearmotor ist bis zu einer Größe von wenigen Millimetern Außendurchmesser problemlos miniaturisierbar.

Die Spule kann wahlweise auf einen Spulenkörper oder auch ohne Spulenkörper gewickelt sein. Sie kann auch mehrteilig sein, d.h. aus mehreren Wicklungen bestehen.

Zur mittelbaren Bestimmung der Position des Läufers sind ein oder zwei Magnetfeldsensoren, hier auch Feldsensoren genannt, vorgesehen. Grundsätzlich kann auch eine höhere Anzahl von Feldsensoren eingesetzt werden. Die Magnetfeldsensoren sind seitlich in axialer Richtung neben der wenigstens einen Wicklung angeordnet. Durch diese Magnetfeldsensoren verläuft in Abhängigkeit von der Position des Läufers ein Anteil des magnetischen Flusses durch die Permanentmagnete. Über die Bestimmung des magnetischen Flusses bzw. der entsprechenden magnetischen Flussdichte B kann auf die Position des Läufers rückgeschlossen werden. Durch einphasige Bestromung der Spulen lässt sich die Ansteuerungselektronik unkompliziert realisieren. Der einfache Aufbau ermöglicht die Miniaturisierung des Antriebs, so dass er sich für den Einsatz in minimalinvasiven Instrumenten eignet. Bevorzugt ist der wenigstens eine Magnetfeldsensor seitlich neben wenigstens einem Statorpolschuh angeordnet. Alternativ kann auch wenigstens ein Magnetfeldsensor in einen Statorpolschuh integriert sein. Besonders vorteilhaft ist die Auswertung der Signaldifferenz von wenigstens zwei Magnetfeldsensoren. Dadurch kann eine stärkere Unabhängigkeit von äußeren Einflüssen wie beispielsweise der Temperatur sowie eine größere Messgenauigkeit erreicht werden.

Die bevorzugte Ausführungsform hat eine Spule und auf beiden Seiten in axialer Richtung Statorpolschuhe, wobei die Spule und die Statorpolschuhe von einem Rückschlusselement umschlossen sind. Seitlich neben den Statorpolschuh bzw. in die Statorpolschuhe integriert sind zwei Magnetfeldsensoren vorgesehen. Der Läufer hat zwei in entgegengesetzter Richtung polarisierte Permanentmagnete, wobei zwischen den beiden Permanentmagneten ein Läuferpolschuh und an den Enden der Permanentmagnete zwei weitere Läuferpolschuhe vorgesehen sind.

Eine Variante des erfindungsgemäßen Linearmotors hat nur einen Statorpolschuh und entsprechend auch nur einen Feldsensor. Passend hierzu kann der Läufer vereinfacht mit nur einem Permanentmagneten und bevorzugt mit zwei Läuferpolschuhen an den Enden des Permanentmagneten ausgestaltet sein.

In einer weiteren Ausgestaltung der Erfindung sind zwei Spulen seitlich in axialer Richtung an zwei Seiten eines Statorpolschuhs angeordnet. Hier ist mindestens ein Magnetfeldsensor, bevorzugt zwei Magnetfeldsensoren in den Statorpolschuh integriert. Bevorzugt wird hier ein Läufer mit zwei entgegengesetzt gepolten Permanentmagneten und jeweils einem Läuferpolschuh zwischen den Permanentmagneten sowie an den äußeren Enden der Permanentmagnete eingesetzt.

Zur Magnetfeldmessung können unterschiedliche Sensortypen zum Einsatz kommen. Die gängigsten Sensoren sind Hall-Effekt-Sensoren, GMR-Sensoren und AMR-Sensoren.

Bei dem erfindungsgemäßen Linearmotor ist kein Gleichgewichtszustand zwischen Reluktanzkraft- und Lorentzkraft für die Positionierung, wie bei den aus dem Stand der Technik bekannten Linearantrieben notwendig. Dies führt im Vergleich zu deutlich höheren Antriebs- und Stellkräften bei gleicher elektrischer Leistung. Dadurch lässt er sich auch in chirurgischen Instrumenten einsetzen.

Die verschiedenen Varianten des erfindungsgemäßen Linearmotors können auch mit einem eisenlosen Stator realisiert werden. Hierbei würden dann die Statorpolschuhe sowie das Rückschlusselement aus nicht-ferromagnetischem Material bestehen oder gar entfallen. Aufgrund der fehlenden magnetfeldleitenden Materialien im Stator ist die magnetische Flussdichte im magnetischen Kreis geringer. Damit wird auch die antreibende Lorentzkraft durch einen elektrischen Stromfluss in der Spule geringer.

In einer weiteren vorteilhaften Ausgestaltung der Erfindung ist zwischen dem Stator und dem Läufer eine Gleitschicht. Diese kann insbesondere im Falle einer rotationssymmetrischen Anordnung als Gleithülse ausgeführt sein. Um die Magnetfelder möglichst wenig zu beeinflussen soll die Gleitschicht aus einem nicht magnetfeldführenden Material, insbesondere aus einem nicht- ferromagnetischen Material bestehen. Ihre Oberfläche umfasst bevorzugt ein Material mit niedrigem Reibungskoeffizienten, beispielsweise PTFE (Polytetrafluorethylen), Siliziumnitrid, Siliziumkarbid, Poly-para-Xylylen Polymere oder DLC (diamond like carbon) wie beispielsweise in der US 5,478,650 offenbart.

Die Gleitschicht kann Unebenheiten auf der dem Läufer zugewandten Seite des Stators ausgleichen.

Der beschriebene Linearmotor kann in einer alternativen Ausführungsform mit einem ebenen, z.B. plattenförmig aufgebauten Stator und einem ebenfalls ebenen bzw. plattenförmigen Polschuh des Läufers realisiert werden. Alternativ können auch mehrere um einen Zylinder oder einen vieleckigen Körper angeordnete Linearmotoren vorgesehen sein. So ergibt sich beispielsweise bei einer gleichmäßigen Anordnung von Linearmotoren um einen Zylinder eine stabile Führung.

Ein erfindungsgemäßer Linearmotor kann auch aus Vollmaterial bestehen und an einem Ende einen Stößel zur Nanopositionierung von Instrumenten aufweisen. Eine solche Vorrichtung ist bevorzugt in der Molekularbiologie, der Mikroelektronik oder auch der Neurochirurgie einsetzbar.

Durch den einfachen Aufbau lässt sich der Antrieb in Bezug auf die axiale Baulänge sehr kompakt ausführen. Damit ist der Antrieb als Ausführung mit einem Hohlläufer für den Einsatz in optischen Systemen gut geeignet.

Der erfindungsgemäße Antrieb kann direkt in das Objektiv einer Endoskopkamera integriert werden. Eine geregelte Einstellung von Zoom- und Fokusfunktion mit Messung der Position der beweglichen optischen Linsengruppen ist damit möglich. Durch die kleine Bauform können nun auch platzsparend Stereokameras mit zwei Einzelobjektiven für 3D-Systeme in ein konventionelles Kameragehäuse integriert werden. Der erfindungsgemäße Linearantrieb ist bevorzugt zweimal in identischer Bauweise für die Bewegung von Linsengruppen entlang der optischen Achse integriert. Durch die geregelte Positionierung der Linsengruppen durch die Linearantriebe können die auf die Bildaufnehmer auftreffenden Bilder scharf angezeigt werden.

In Chip-on-the-Tip-Objektiven kann die komplette Optik einschließlich eines Kamerachips in die Spitze eines Endoskops integriert werden. Dabei werden optische Linsengruppen beispielsweise entlang der optischen Achse bewegt, um eine Scharfeinstellung (Fokussierung) oder eine Vergrößerung (Zoomfunktion) des Bilds zu ermöglichen. Der erfindungsgemäße geregelte Linearantrieb kann in miniaturisierter Form ebenfalls in Videoendoskopen zum Einsatz kommen. Durch den erfindungsgemäßen Antrieb bietet sich dabei die Möglichkeit, die Position der Linsengruppen durch Rückkopplung der Positionssignale zu regeln. Durch die Messung der Läuferposition mit den Magnetfeldsensoren ist die Position der Linsengruppen bekannt. Bei einem Antrieb für die Fokusverstellung kann damit bei Kenntnis über das optische System der Objektabstand zu scharfgestellten Objekten ermittelt werden. Mit dem erfindungsgemäßen Linearantrieb ist auch eine einfache Kopplung von zwei oder mehreren beweglichen Linsengruppen möglich. Bei Stereoendoskopen in Chip-on-the-Tip-Ausführung werden für die Paarbildaufnahme häufig zwei nebeneinander liegende optische Linsensysteme an der Endoskopspitze verwendet. Sollen hierbei jeweils Linsengruppen für eine Fokussierung oder Zoomfunktion axial motorisch beweglich sein, können zwei erfindungsgemäße Antriebe dafür eingesetzt werden.

Durch den Einsatz des erfindungsgemäßen Antriebs in einem chirurgischen Instrument ist es möglich, einen Öffnungswinkel Phi eines Maulteils geregelt einzustellen. Über die Magnetfeldsensoren kann der Läufer des erfindungsgemäßen Antriebs wie oben beschrieben innerhalb des Stators positioniert werden. Wird eine Zug-Druck-Stange, die durch eine Linearbewegung das Öffnen und Schließen eines Maulteils hervorruft, an den Antrieb montiert, kann über die Position des Läufers der Öffnungswinkel des Instruments Phi eingestellt werden. Durch den geregelten Betrieb kann unabhängig von der Schließkraft des Maulteils der Öffnungswinkel Phi beibehalten oder eingestellt werden. Da es sich bei dem erfindungsgemäßen Antrieb um einen über Lorentzkraft betätigten Linearmotor handelt, kann über den Zusammenhang der Stromstärke in der Spule auch die axial wirkende Antriebskraft ermittelt werden. Dadurch kann auch die Schließkraft am Maulteil berechnet werden. Dieser Zusammenhang spielt vor allem bei neuartigen Force-Feedback-Systemen eine große Rolle, bei denen der operierende Arzt eine Rückmeldung über die Höhe der Schließkraft am Maulteil erhält.

### Beschreibung der Zeichnungen

Die Erfindung wird nachstehend ohne Beschränkung des allgemeinen Erfindungsgedankens anhand von Ausführungsbeispielen unter Bezugnahme auf die Zeichnungen exemplarisch beschrieben.
- Figur 1: zeigt schematisch einen Linearmotor entsprechend der Erfindung in einem Längsschnitt.
- Figur 2: zeigt den linearen Motor in einem Querschnitt.
- Figur 3: zeigt den magnetischen Feldverlauf in einer ersten Läuferposition.
- Figur 4: zeigt den magnetischen Feldverlauf in einer zweiten Läuferposition.
- Figur 5: zeigt den magnetischen Feldverlauf in einer dritten Läuferposition.
- Figur 6: zeigt eine weitere Ausführungsform mit nur einem Statorpolschuh.
- Figur 7: zeigt eine weitere Ausführungsform mit zwei Spulen.
- Figur 8: zeigt die Sensorsignale einer Anordnung nach Figur 1 in Abhängigkeit von der Position.
- Figur 9: zeigt die Differenz der Sensorsignale in Abhängigkeit von der Position.
- Figur 10: zeigt die Sensorsignale in einer Anordnung nach Figur 6.
- Figur 11: zeigt ein Endoskop mit einem durch einen linearen Motor schwenkbaren Prisma.

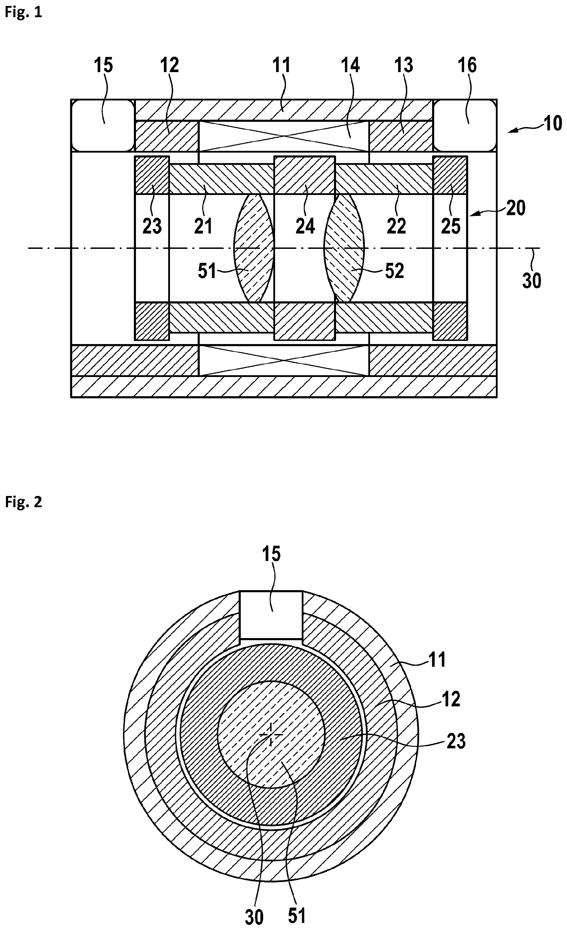

Die Figur 1 zeigt schematisch einen Linearmotor entsprechend der Erfindung in einem Längsschnitt. Entsprechend dem hier dargestellten bevorzugten Ausführungsbeispiel ist der Linearmotor überwiegend rotationssymmetrisch um eine Mittelachse 30 aufgebaut. Er umfasst einen Stator 10 sowie einen Läufer 20.

Der Stator 10 hat eine Spule 14, welche von einem vorzugsweise zylindrischen Rückschlusselement 11 in radialer Richtung umschlossen ist. In axialer Richtung ist die Spule 14 durch einen ersten Statorpolschuh 12 und einen zweiten Statorpolschuh 13 eingeschlossen. Bevorzugt sind auch diese Statorpolschuhe durch das Rückschlusselement in radialer Richtung umschlossen. Weiterhin ist ein erster Feldsensor 15 auf der Seite des ersten Statorpolschuhs 12 in axialer Richtung neben der Spule und ein zweiter Feldsensor 16 auf der Seite des zweiten Statorpolschuhs 13 in axialer Richtung neben der Spule 14 angeordnet. Bevorzugt ist wenigstens ein Feldsensor in einem Ausschnitt eines Statorpolschuhs angeordnet. Besonders bevorzugt erstreckt sich der Ausschnitt, wie in dieser Figur dargestellt, in axialer Richtung, er kann sich aber auch in radialer Richtung erstrecken. Der Ausschnitt kann auch bis in das Rückschlusselement 11 fortgesetzt sein, um einem größeren Feldsensor hinreichend Einbauraum zu bieten. Bevorzugt sind die Statorpolschuhe und/oder das Rückschlusselement ringförmig ausgebildet.

Der Läufer hat einen ersten Permanentmagnet 21 und einen zweiten Permanentmagnet 22, welche in entgegengesetzter Richtung polarisiert sind. Zwischen den beiden Permanentmagneten ist ein mittlerer Läuferpolschuh 24 angeordnet. An den in axialer Richtung dem mittleren Läuferpolschuh 24 entgegengesetzten Seiten sind zum ersten Permanentmagnet 21 hin ein erster äußerer Läuferpolschuh 23 und zum zweiten Permanentmagnet 22 hin ein zweiter äußerer Läuferpolschuh 25 angeordnet. Der Läufer ist bevorzugt hohl, besonders bevorzugt hohlzylindrisch ausgeführt. Bevorzugterweise sind die Permanentmagnete und/oder die Läuferpolschuhe ringförmig ausgebildet.

Bevorzugt sind die beiden Feldsensoren 15, 16 in derselben durch die Mittelachse 30 verlaufenden Schnittebene angeordnet, sie können aber auch in anderen Ebenen angeordnet sein.

Der Antrieb ist bevorzugt so ausgelegt, dass innerhalb des Verfahrwegs des Läufers 10 im unbestromten Zustand keine axial gerichteten Reluktanzkräfte herrschen. Wird die Spule 14 des Antriebs mit einem elektrischen Strom versorgt, entsteht eine Lorentzkraft, die auf den Läufer unabhängig von dessen Position einwirkt.

Grundsätzlich kann diese Ausführungsform wie auch die anderen in diesem Dokument vorgestellten Ausführungsformen mit einem eisenlosen Stator realisiert werden. Hierbei würden dann die Statorpolschuhe 12, 13 sowie das Rückschlusselement 11 aus nicht-ferromagnetischem Material bestehen oder gar entfallen. Aufgrund der fehlenden magnetfeldleitenden Materialien im Stator ist die magnetische Flussdichte im magnetischen Kreis geringer. Damit wird auch die antreibende Lorentzkraft durch einen elektrischen Stromfluss in der Spule geringer.

In der Figur 2 ist eine Ansicht in Richtung der Mittelachse 30 dargestellt. Hier ist die bevorzugte konzentrische Anordnung der Komponenten deutlich zu erkennen. In dieser Ausführungsform ist der erste Feldsensor 15 in einem Ausschnitt, der durch das Rückschlusselement 11 in den ersten Statorpolschuh 12 geht, angeordnet.

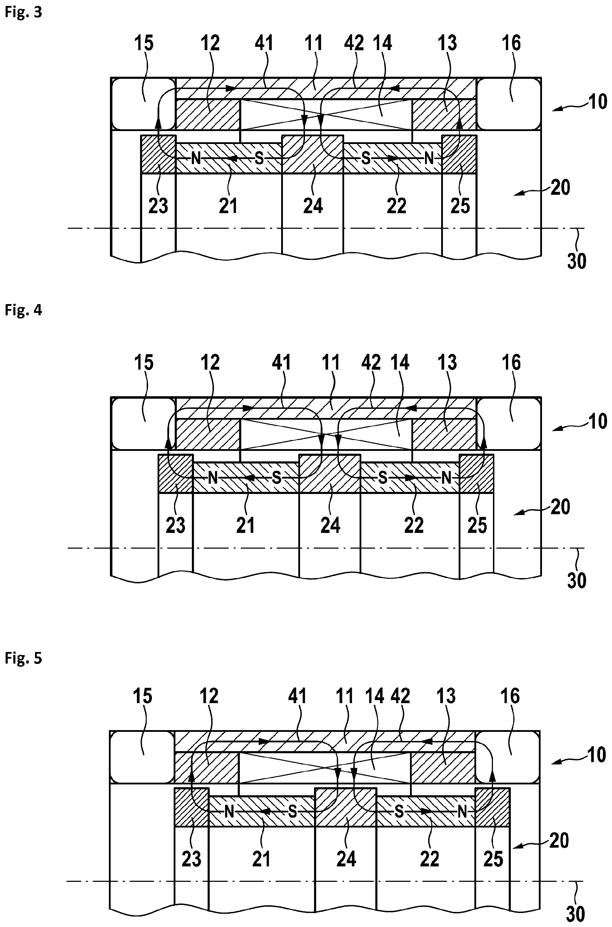

Die Figuren 3, 4 und 5 zeigen die magnetischen Feldverläufe bei verschiedenen Positionen des Läufers 20 relativ zum Stator 10. In der Figur 3 ist der Läufer 20 gegenüber der Mittelposition, welche in der Figur 4 dargestellt ist, nach links verschoben, während er in der Figur 5 nach rechts verschoben ist. Grundsätzlich ergibt sich ein erster magnetischer Kreis 41 ausgehend von dem ersten Permanentmagnet 21. Das Feld verläuft durch den ersten äußeren Läuferpolschuh 23 über den ersten Feldsensor 15 bzw. den ersten Statorpolschuh 12 weiter durch das Rückschlusselement 11, über die Spule 14 weiter in den mittleren Läuferpolschuh 24 und zurück zum ersten Permanentmagnet 21. Entsprechend verläuft der zweite magnetische Kreis 42 in umgekehrter Richtung.

Zur Darstellung des magnetischen Feldverlaufs sind symbolisch die Linien 41 und 42 eingezeichnet. Tatsächlich verteilt sich das magnetische Feld beispielsweise über die gesamte Stirnfläche der Permanentmagnete 21, 22. Ebenso tritt das magnetische Feld in radialer Richtung aus den Läuferpolschuhen verteilt über die Fläche aus.

In der Darstellung der Figur 3 ist offensichtlich, dass ein überwiegender Teil des magnetischen Felds durch den ersten Feldsensor 15 verläuft, während ein vernachlässigbarer Anteil durch den ersten Statorpolschuh 12 geht. Das magnetische Feld ausgehend vom zweiten Läuferpolschuh 25 tritt überwiegend über den zweiten Statorpolschuh 13 in das Rückschlusselement 11 ein. Es wird nur ein minimaler und daher vernachlässigbarer Anteil durch den zweiten Feldsensor 16 hindurch treten.

In der Figur 4 tritt ein Teil des magnetischen Feldes aus dem ersten Läuferpolschuh 23 über den ersten Feldsensor 15 und parallel hierzu über den ersten Statorpolschuh 12 in das Rückschlusselement 11 ein. Ähnliches gilt für das Feld aus dem zweiten Läuferpolschuh 25. Hier teilt sich das Feld ebenfalls zwischen dem zweiten Feldsensor 16 und dem zweiten Statorpolschuh 13 auf.

In der Figur 5 tritt der überwiegende Teil des Feldes aus dem ersten Läuferpolschuh 23 über den ersten Statorpolschuh 12 in das Rückschlusselement 11 ein, während der Großteil des Feldes aus dem zweiten Läuferpolschuh 25 durch den zweiten Feldsensor 16 verläuft.

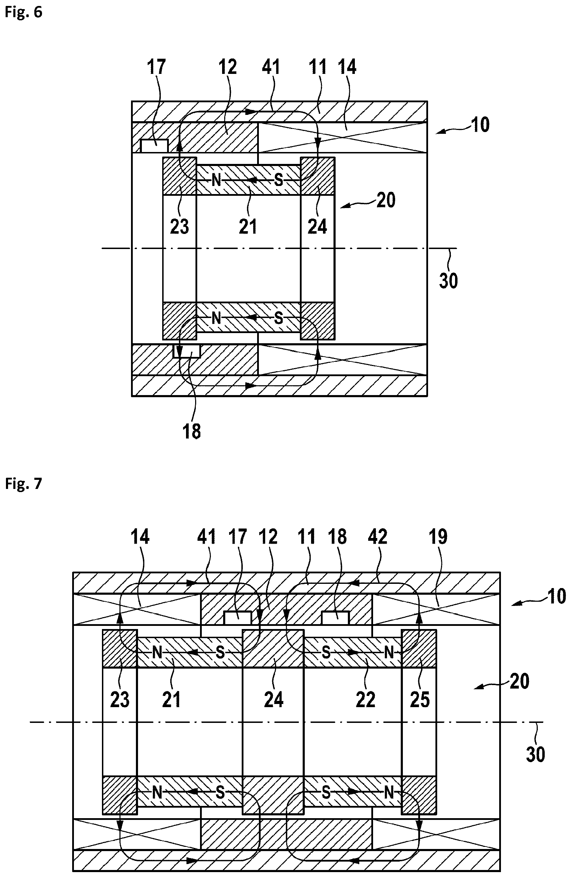

In der Figur 6 ist eine weitere Ausführungsform der Erfindung dargestellt. In dieser Ausführungsform hat der Stator nur einen Statorpolschuh 12, und der Läufer hat nur einen Permanentmagneten 21. Es ist ein erster Feldsensor 17 und optional ein zweiter Feldsensor 18 vorgesehen. Es werden hier bevorzugt integrierte Feldsensoren 17, 18 mit einer kleineren Bauform verwendet, die in den ersten Statorpolschuh 12 integriert werden können. Bevorzugt werden diese in eine Aussparung des ersten Statorpolschuhs 12 eingesetzt. Die Feldsensoren 17, 18 sind in axialer Richtung gegeneinander versetzt. Sie können bevorzugt auch auf verschiedenen Schnittebenen durch die Rotationsachse angeordnet sein. In dem hier gezeigten Ausführungsbeispiel sind sie in der gleichen Ebene, auf unterschiedlichen Seiten der Rotationsachse angeordnet. In Abhängigkeit von der Position des Läufers 20 in Bezug auf den Stator 10 durchsetzt der magnetische Fluss ausgehend von dem ersten äußeren Läuferpolschuh 23 einen der Feldsensoren. In der dargestellten Position verläuft ein größerer Teil des magnetischen Flusses durch den zweiten Feldsensor 18, während sich der erste Feldsensor im feldfreien Raum befindet.

Durch die hier dargestellte Anordnung kann der Magnetfeldsensor nur im Magnetfeld eines Polschuhs liegen. Dadurch können einfachere Magnetfeldsensoren eingesetzt werden, die ein Ausgangssignal unabhängig von der Richtung des Magnetfelds liefern. Solche Sensoren sind beispielsweise GMR-(Giant Magneto Resistance) Sensoren. Beim Stand der Technik ist es oftmals notwendig, richtungssensitive Magnetfeldsensoren wie beispielsweise Hall-Sensoren einzusetzen, um eine eindeutige Positionserkennung zu erreichen. Solche Sensoren sind meist größer, teurer und erfordern eine komplexere Ansteuerung bzw. Auswertung.

Die Integration der Magnetfeldsensoren in den Statorpolschuh erlaubt insbesondere bei Miniaturmotoren eine wesentlich bessere Raumausnutzung. Diese Ausführungsform ist gleichzeitig robuster, da die Magnetfeldsensoren durch den Statorpolschuh mechanisch geschützt werden. Dadurch kann auf ein separates Gehäuse der Magnetfeldsensoren verzichtet werden.

Grundsätzlich kann bei dieser Ausführungsform eines Linearmotors auch eine Anordnung aus einem ersten Magnetfeldsensor 15 neben einem ersten Statorpolschuh 12 wie in der Ausführungsform nach Figur 1 eingesetzt werden. Ebenso kann auch die Ausführungsform der Figur 1 mit einem in den ersten Statorpolschuh 12 integrierten ersten Feldsensor 17, entsprechend diesem Ausführungsbeispiel realisiert werden. Bevorzugt wird dann auch der zweite Feldsensor 16 aus dem Ausführungsbeispiel der Figur 1 durch einen in den zweiten Statorpolschuh 13 integrierten zweiten Feldsensor 18 ersetzt.

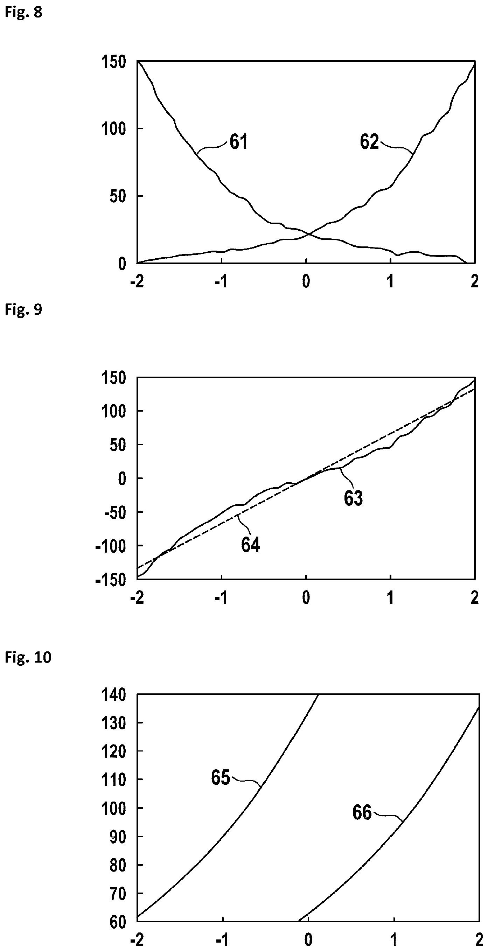

In der Figur 7 ist eine weitere Ausführungsform entsprechend der Erfindung dargestellt. Der Läufer 20 entspricht dem Läufer aus der ersten Ausführungsform, wie sie beispielsweise in der Figur 1 dargestellt ist. Der Stator hat hier eine erste Spule 14 und eine zweite Spule 19. Die Spulen können einphasig (identische Stromstärke) oder zweiphasig (unterschiedliche Stromstärken in den beiden Spulen) versorgt werden. Zwischen den beiden Spulen ist ein Statorpolschuh 12 angeordnet. Die Spulen und der Statorpolschuh werden durch das Rückschlusselement 11 umschlossen. Die Feldsensoren 17, 18 sind in axialer Richtung gegeneinander versetzt in den Statorpolschuh 12 integriert bzw. in Aussparungen des Statorpolschuhs aufgenommen. Das Magnetfeld aus dem mittleren Läuferpolschuh 24 läuft je nach Position des Läufers ganz oder teilweise durch den ersten Feldsensor 17 oder den zweiten Feldsensor 18. Die Ausgangssignale der Feldsensoren 17, 18 entsprechen näherungsweise ebenfalls den Kurven 61 und 62 aus der Figur 8. Auch hier kann durch eine Differenzbildung ein Signal entsprechend der Figur 9 erreicht werden.

Die Figur 8 zeigt den Signalverlauf der Feldsensoren beispielsweise entsprechend den Darstellungen aus den Figuren 3, 4 und 5. Das Diagramm zeigt auf der horizontalen Achse den Abstand in Millimetern bezogen auf die Nullposition "0", welche beispielsweise die Figur 4 entspricht. Eine Auslenkung von -2 mm entspricht der Figur 3 und eine Auslenkung von +2 mm entspricht der Figur 5. An der vertikalen Achse sind die Amplituden der Sensorsignale von 0-150 skaliert aufgetragen. Die Kurve 61 zeigt das Signal des ersten Feldsensors 15, während die Kurve 62 den Verlauf des Signals des zweiten Feldsensors 16 zeigt.

Die Kurve 61 zeigt links, bei einer Auslenkung von -2 mm die maximale Amplitude, was der maximalen magnetischen Flussdichte durch den ersten Feldsensor 15 entspricht. Diese wird bei der in Figur 3 dargestellten Lage des Läufers erreicht. Gleichzeitig befindet sich der zweite Feldsensor 16 im nahezu feldfreien Raum, so dass das Sensorsignale entsprechend der Kurve 62 nahezu null ist. In der Mittelposition entsprechend der Figur 4 ist die Flussdichte bei beiden Feldsensoren näherungsweise gleich, so dass auch die beiden Kurven 61 und 62 an der Position "0" die gleiche Amplitude haben. An der rechten Position bei +2 mm verhalten sich die Sensorsignale umgekehrt wie an der linken Position. Hier geht die maximale magnetische Flussdichte durch den zweiten Feldsensor 16, während der erste Feldsensor 15 im nahezu feldfreien Raum liegt.

Die Figur 9 zeigt die Summe der Kurven 61 und 62 aus der Figur 8 in der Kurve 63. Diese Kurve kann durch eine lineare Approximation 64 gut angenähert werden. Es ergibt sich somit ein Messsignal, welches mit geringen Abweichungen proportional zur Position des Läufers ist. Dieses Messsignal kann auch in eine Regelschleife eingespeist werden, so dass die Position des Läufers in Abhängigkeit von einem Sollwert konstant gehalten werden kann.

Die Figur 10 zeigt die Signale der Feldsensoren in einer Anordnung entsprechend der Figur 6. Auch hier zeigt die horizontale Achse die Verschiebung in Richtung der Mittelachse an, während die vertikale Achse die Amplitude der Sensorsignale angibt. Die Kurve 65 gibt die Signalamplitude des ersten Feldsensors 17 an, während die zweite Kurve 66 die Amplitude des zweiten Feldsensors 18 angibt. So kann über die Auswertung, welcher Sensor ein Signal abgibt in Kombination mit der Signalamplitude des Sensors die exakte Position ermittelt werden. In diesem Beispiel sind nur zwei Sensoren dargestellt. Selbst verständlich kann hier eine beliebige höhere Anzahl von Feldsensoren verwendet werden, um die Auflösung und/oder die maximale Weglänge zu erhöhen.

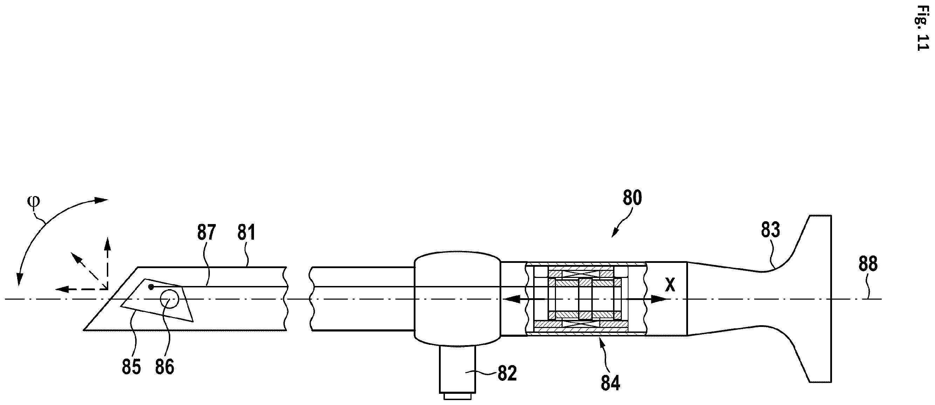

Die Figur 11 zeigt ein Endoskop 80, bei dem ein Linearmotor 84 entsprechend der Erfindung zur Verstellung des Blickwinkels ϕ eingesetzt wird. Das Endoskop hat einen distalen Schaft 81 sowie ein proximales Okular 83. Es können optionale Anschlüsse 82 zur Lichteinspeisung oder auch zur Einspeisung von Flüssigkeiten und/oder Gasen vorgesehen sein. Das Endoskop hat eine Achse 88, welche bevorzugt auch die optische und/oder mechanische Achse ist. Am distalen Ende des Schafts 81 ist ein Prisma 85 um eine Lagerung 86 schwenkbar angeordnet. Das Prisma ermöglicht eine Ablenkung des optischen Strahlengangs, so dass unter verschiedenen Blickwinkeln ϕ in das distale Ende des Endoskops eintretendes Licht erfasst werden kann. Eine Verstellung des Prismas erfolgt mittels eines Linearmotors 84 über eine Schub/Zugstange 87.

### Bezugszeichenliste

- 10: Stator
- 11: Rückschlußelement
- 12: Erster Statorpolschuh
- 13: Zweiter Statorpolschuh
- 14: Spule
- 15: erster Feldsensor
- 16: zweiter Feldsensor
- 17: erster integrierter Feldsensor
- 18: zweiter integrierter Feldsensor
- 19: Zweite Spule
- 20: Läufer
- 21: erster Permanentmagnet
- 22: zweiter Permanentmagnet
- 23: erster Läuferpolschuh
- 24: zweiter Läuferpolschuh
- 25: dritter Läuferpolschuh
- 30: Mittelachse
- 41: erster Feldverlauf
- 42: zweiter Feldverlauf
- 51: erste Linse
- 52: zweite Linse
- 61: Signalverlauf erster Feldsensor
- 62: Signalverlauf zweiter Feldsensor
- 63: Differenz der Signalverläufe
- 64: lineare Approximation
- 65: Signalverlauf erster integrierter Feldsensor
- 66: Signalverlauf zweiter integrierter Feldsensor
- 80: Endoskop
- 81: Endoskopschaft
- 82: Anschluss
- 83: Okular
- 84: Linearmotor
- 85: Prisma
- 86: Lager
- 87: Schub/Zugstange
- 88: Mittelachse

## Patentansprüche

1. Linearmotor umfassend
einen Stator (10) mit einer Mittelachse (30), einer Spule (14), die in Axialrichtung zur Mittelachse (30) auf zwei Seiten von einem ersten ringförmigen Statorpolschuh (12) und einem zweiten ringförmigen Statorpolschuh (13) eingeschlossen ist, einem Rückschlusselement (11), welches in radialer Richtung die Spule (14) umschließt, sowie einem ersten Feldsensor (15), in Axialrichtung neben der Spule (14) auf der Seite des ersten Statorpolschuhs (12) und einem zweiten Feldsensor (16) in Axialrichtung neben der Spule (14) auf der Seite des zweiten Statorpolschuhs (13), und
einen in Axialrichtung im Stator verschiebbaren, hohlen Läufer (20) mit einem ersten ringförmigen Permanentmagneten (21) und einem zweiten ringförmigen Permanentmagneten (22), die in entgegengesetzter Richtung polarisiert sind, einem ringförmigen mittleren Läuferpolschuh (24) zwischen den beiden Permanentmagneten sowie einem ringförmigen ersten äußeren Läuferpolschuh (23) an der in axialer Richtung dem mittleren Läuferpolschuh (24) entgegengesetzten Seite des ersten Permanentmagneten (21) und einem ringförmigen zweiten äußeren Läuferpolschuh (25) an der in axialer Richtung dem mittleren Läuferpolschuh (24) entgegengesetzten Seite des zweiten Permanentmagneten (22),
wobei wenigstens ein Teil des magnetischen Flusses von dem ersten äußeren Läuferpolschuh (23) und/oder dem zweiten äußeren Läuferpolschuh (25) durch wenigstens einen der Feldsensoren (15,16) verläuft.

2. Linearmotor umfassend
einen Stator (10) mit einer Mittelachse (30), einer Spule (14), die in Axialrichtung zur Mittelachse (30) auf einer Seite von einem ringförmigen Statorpolschuh (12) abgeschlossen ist, einem Rückschlusselement (11), welches in radialer Richtung die Spule (14) umschließt, sowie einem Feldsensor (17), in den ersten Statorpolschuh (12) integriert, und
einen in Axialrichtung im Stator verschiebbaren, hohlen Läufer (20) mit einem ersten ringförmigen Permanentmagneten (21), sowie einem ringförmigen ersten äußeren Läuferpolschuh (23) und einem ringförmigen zweiten äußeren Läuferpolschuh (24) auf den axial gegenüberliegenden Seiten des Permanentmagneten (21),
wobei wenigstens ein Teil des magnetischen Flusses von dem ersten äußeren Läuferpolschuh (23) durch den Feldsensor (17) verläuft.

3. Linearmotor nach Anspruch 2,
**dadurch gekennzeichnet, dass**
ein zweiter Feldsensor (18) in den Statorpolschuh (12) integriert und in Axialrichtung gegen den ersten Feldsensor (17) versetzt ist.

4. Linearmotor umfassend
einen Stator (10) mit einer Mittelachse (30), einer ersten Spule (14), einer zweiten Spule (19), einem ersten ringförmigen Statorpolschuh (12) zwischen den beiden Spulen (14, 19), einem Rückschlusselement (11), welches in radialer Richtung die Spulen (14, 19) umschließt, sowie einem ersten Feldsensor (17) und einem zweiten Feldsensor (18), wobei beide Feldsensoren (17, 18) in den Statorpolschuh (12) integriert und in Axialrichtung zur Mittelachse (30) gegeneinander versetzt sind, und
einen in Axialrichtung im Stator verschiebbaren, hohlen Läufer (20) mit einem ersten ringförmigen Permanentmagneten (21) und einem zweiten ringförmigen Permanentmagneten (22), die in entgegengesetzter Richtung polarisiert sind, einem ringförmigen mittleren Läuferpolschuh (24) zwischen den beiden Permanentmagneten sowie einem ringförmigen ersten äußeren Läuferpolschuh (23) an der in axialer Richtung dem mittleren Läuferpolschuh (24) entgegengesetzten Seite des ersten Permanentmagneten (21) und einem ringförmigen zweiten äußeren Läuferpolschuh (25) an der in axialer Richtung dem mittleren Läuferpolschuh (24) entgegengesetzten Seite des zweiten Permanentmagneten (22),
wobei wenigstens ein Teil des magnetischen Flusses von dem mittleren Läuferpolschuh (24) durch wenigstens einen der Feldsensoren (17,18) verläuft.

5. Linearmotor nach einem der vorhergehenden Ansprüche,
**dadurch gekennzeichnet, dass**
wenigstens einer der Feldsensoren (15, 16, 17, 18) in einem Ausschnitt des jeweiligen Statorpolschuhs (12, 13) angeordnet oder in den Statorpolschuh integriert ist.

6. Linearmotor nach Anspruch 1, 3 oder 4,
**dadurch gekennzeichnet, dass**
eine Auswerteschaltung vorgesehen ist, welche ein Signal zur Positionsangabe des Läufers mithilfe der Summe aus den Signalen der beiden Feldsensoren (15, 16 oder 17,18) erzeugt.

7. Linearmotor nach einem der vorhergehenden Ansprüche,
**dadurch gekennzeichnet, dass**
eine Regelschaltung vorgesehen ist, welche eine Sollgröße für eine Position des Läufers mit einem Messwert aus wenigstens einem Signal wenigstens eines Feldsensors vergleicht und ein Stellsignal zur Konstanthaltung der Position des Läufers erzeugt.

8. Linearmotor nach einem der vorhergehenden Ansprüche,
**dadurch gekennzeichnet, dass**
wenigstens einer der Feldsensoren (15, 16) in radialer Richtung von dem Rückschlusselement (11) umschlossen ist.

9. Linearmotor nach einem der vorhergehenden Ansprüche,
**dadurch gekennzeichnet, dass**
wenigstens ein Statorpolschuh (12,13) und/oder wenigstens ein Läuferpolschuh (23, 24, 25) und/oder das Rückschlusselement (11) wenigstens ein ferromagnetisches Material umfassen.

10. Linearmotor nach einem der vorhergehenden Ansprüche,
**dadurch gekennzeichnet, dass**
im Inneren des Läufers (20) ein optisches Element (51, 52) aufgenommen werden kann.

11. Linearmotor nach einem der vorhergehenden Ansprüche,
**dadurch gekennzeichnet, dass**
eine Gleithülse mit einem nicht ferromagnetischen Material mit niedrigem Reibungskoeffizienten an der Oberfläche zwischen Stator (10) und Läufer (20) angeordnet ist.

12. Kamera mit einem Linearmotor nach einem der vorhergehenden Ansprüche,
**dadurch gekennzeichnet, dass**
der Linearmotor zur Steuerung wenigstens einer optischen Komponente vorgesehen ist.

13. Endoskop mit einem Linearmotor nach einem der vorhergehenden Ansprüche,
**dadurch gekennzeichnet, dass**
der Linearmotor zur Steuerung wenigstens einer optischen Komponente vorgesehen ist.

14. Medizinisches Instrument nach einem der vorhergehenden Ansprüche, **dadurch gekennzeichnet, dass**
der Linearmotor zur Einstellung des Öffnungswinkels eines Maulteils vorgesehen ist.

## Claims

1. Linear motor comprising
a stator (10) having a middle axis (30), a coil (14) which is enclosed in the axial direction to the middle axis (30) at two sides by a first annular stator pole shoe (12) and a second annular stator pole shoe (13), a magnetic circuit closing element (11) which encloses the coil (14) in a radial direction, as well as a first field sensor (15) arranged in the axial direction next to the coil (14) at the side of the first stator pole shoe (12) and a second field sensor (16) arranged in the axial direction next to the coil (14) at the side of the second stator pole shoe (13), and
a hollow rotor (20) being displaceable in the axial direction in the stator, the rotor comprising a first annular permanent magnet (21) and a second annular permanent magnet (22) which are polarized in opposite directions, an annular middle rotor pole shoe (24) between the two permanent magnets as well as an annular first outer rotor pole shoe (23) at the side of the first permanent magnet (21) opposite in the axial direction to the middle rotor pole shoe (24) and an annular second outer rotor pole shoe (25) at a side of the second permanent magnet (22) opposite in the axial direction to the middle rotor pole shoe (24),
wherein at least a portion of the magnetic flow from the first outer rotor pole shoe (23) and/or the second outer rotor pole shoe (25) flows through at least one of the field sensors (15, 16).

2. Linear motor comprising
a stator (10) having a middle axis (30), a coil (14) which is enclosed in the axial direction to the middle axis (30) at one side by an annular stator pole shoe (12), a magnetic circuit closing element (11) which encloses the coil (14) in a radial direction, as well as a field sensor (17) integrated into the stator pole shoe (12), and
a hollow rotor (20) being displaceable in the axial direction in the stator, the rotor comprising a first annular permanent magnet (21) as well as an annular first outer rotor pole shoe (23) and an annular second outer rotor pole shoe (24) at the axially opposed sides of the permanent magnet (21),
wherein at least a portion of the magnetic flow from the first outer rotor pole shoe (23) flows through the field sensor (17).

3. Linear motor according to claim 2, **characterized in that** a second field sensor (18) is integrated into the stator pole shoe (12) and is displaced in the axial direction in relation to the first field sensor (17).

4. Linear motor comprising
a stator (10) having a middle axis (30), a first coil (14), a second coil (19), a first annular stator pole shoe (12) between the two coils (14, 19), a magnetic circuit closing element (11) which encloses the coils (14, 19) in a radial direction, as well as a first field sensor (17) and a second field sensor (18), wherein both field sensors (17, 18) are integrated into the stator pole shoe (12) and are displaced in an axial direction to the middle axis (30) in relation to one another, and
a hollow rotor (20) being displaceable in the axial direction in the stator, the rotor comprising a first annular permanent magnet (21) and a second annular permanent magnet (22) which are polarized in opposite directions, an annular middle rotor pole shoe (24) between the two permanent magnets, as well as an annular first outer rotor pole shoe (23) at the side of the first permanent magnet (21) opposite in the axial direction to the middle rotor pole shoe (24), and an annular second outer rotor pole shoe (25) at a side of the second permanent magnet (22) opposite in the axial direction to the middle rotor pole shoe (24),
wherein at least a portion of the magnetic flow from the middle rotor pole shoe (24) flows through at least one of the field sensors (17, 18).

5. Linear motor according to any preceding claim, **characterized in that** an at least one of the field sensors (15, 16, 17, 18) is arranged in a cavity of the corresponding stator pole shoe (12, 13) or is integrated into the stator pole shoe.

6. Linear motor according to claim 1, 3 or 4, **characterized in that** an evaluation circuitry is provided which generates a signal to indicate the position of the rotor using the sum of the signals of both field sensors (15, 16 or 17, 18).

7. Linear motor according to any preceding claim, **characterized in that** a control circuitry is provided which compares a target value for a position of the rotor to a measurement value of at least one signal of at least one field sensor and generates a setting signal to keep the position of the rotor constant.

8. Linear motor according to any preceding claim, **characterized in that** at least one of the field sensors (15, 16) is enclosed in the radial direction by the magnetic circuit closing element (11).

9. Linear motor according to any preceding claim, **characterized in that** at least one stator pole shoe (12, 13) and/or at least one rotor pole shoe (23, 24, 25) and/or the magnetic circuit closing element (11) comprise at least one ferromagnetic material.

10. Linear motor according to any preceding claim, **characterized in that** on the inside of the rotor (20) an optical element (51, 52) may be received.

11. Linear motor according to any preceding claim, **characterized in that** a slide sleeve comprising a non-ferromagnetic material with a low coefficient of friction is arranged at the surface between stator (10) and rotor (20).

12. Camera comprising a linear motor according to any preceding claim, **characterized in that** the linear motor is provided for controlling at least one optical component.

13. Endoscope comprising a linear motor according to any preceding claim, **characterized in that** the linear motor is provided for the control of at least one optical component.

14. Medical instrument according to any preceding claim, **characterized in that** the linear motor is provided for setting the opening angle of a jaw element.

## Revendications

1. Moteur linéaire, comprenant
un stator (10) présentant un axe central (30), une bobine (14) qui est enserrée des deux côtés par une première pièce polaire de stator annulaire (12) et une deuxième pièce polaire de stator annulaire (13), un élément de fermeture arrière (11) qui entoure la bobine (14) en direction radiale, ainsi qu'un premier capteur de champ (15), adjacent en direction radiale à la bobine (14) sur la première pièce polaire de stator (12) et un deuxième capteur de champ (16), adjacent en direction radiale à la bobine (14) sur le côté de la deuxième pièce polaire de stator (13), et
un rotor creux (20) pouvant être déplacé en direction axiale dans le stator, comportant un premier aimant permanent annulaire (21) et un deuxième aimant permanent annulaire (22), qui sont polarisés en sens opposé, une pièce polaire de rotor centrale annulaire (24) entre les deux aimants permanents ainsi qu'une première pièce polaire de rotor extérieure annulaire (23) sur le côté du premier aimant permanent (21), qui est opposé en direction axiale à la pièce polaire de rotor centrale (24), et une deuxième pièce polaire de rotor extérieure annulaire (25) sur le côté du deuxième aimant permanent (22), qui est opposé en direction axiale à la pièce polaire de rotor centrale (24),
dans lequel au moins une partie du flux magnétique provient de la première pièce polaire de rotor extérieure (23) et/ou de la deuxième pièce polaire de rotor extérieure (25) et passe par au moins l'un des capteurs de champ (15, 16).

2. Moteur linéaire, comprenant
un stator (10) présentant un axe central (30), une bobine (14) se terminant en direction axiale sur l'axe central (30) sur un côté d'une pièce polaire de stator annulaire (12), un élément de fermeture arrière (11) qui entoure la bobine (14) en direction radiale, ainsi qu'un capteur de champ (17) intégré à la première pièce polaire de stator (12), et un rotor creux (20) pouvant être déplacé en direction axiale dans le stator, comportant un premier aimant permanent annulaire (21) ainsi qu'une première pièce polaire de rotor extérieure annulaire (23) et une deuxième pièce polaire de rotor extérieure annulaire (24) sur les côtés axialement opposés de l'aimant permanent (21),
dans lequel au moins une partie du flux magnétique provient de la première pièce polaire de rotor extérieure (23) et passe par le capteur de champ (17).

3. Moteur linéaire selon la revendication 2,
**caractérisé en ce qu'**un deuxième capteur de champ (18) est intégré à la pièce polaire de stator (21) et est décalé en direction radiale contre le premier capteur de champ (17).

4. Moteur linéaire, comprenant
un stator (10) présentant un axe central (30), une première bobine (14), une deuxième bobine (19), une première pièce polaire de stator annulaire (12) entre les deux bobines (14, 19), un élément de fermeture arrière (11) qui entoure en direction radiale les bobines (14, 19), ainsi qu'un premier capteur de champ (17) et un deuxième capteur de champ (18), dans lequel les deux capteurs de champ (17, 18) sont intégrés à la pièce polaire de stator (12) et sont décalés l'un par rapport à l'autre en direction radiale vers l'axe central (30), et
un rotor creux (20) pouvant être déplacé en direction axiale dans le rotor, comportant un premier aimant permanent annulaire (21) et un deuxième aimant permanent annulaire (22), qui sont polarisés en sens opposé, une pièce polaire de rotor centrale annulaire (24) entre les deux aimants permanents ainsi qu'une première pièce polaire de rotor extérieure annulaire (21) sur le côté du premier aimant permanent (21), qui est opposé en direction radiale à la pièce polaire de rotor centrale (24), et une deuxième pièce polaire de rotor extérieure annulaire (25) sur le côté du deuxième aimant permanent (22), qui est opposé en direction axiale à la pièce polaire de rotor centrale (24),
dans lequel au moins une partie du flux magnétique va de la première pièce polaire de rotor extérieure (24) en passant par au moins l'un des capteurs de champ (17, 18).

5. Moteur linéaire selon l'une quelconque des revendications précédentes, **caractérisé en ce qu'**au moins l'un des capteurs de champ (15, 16, 17, 18) est disposé dans une partie de la pièce polaire de stator (12, 13) respective ou est intégré à la pièce polaire de stator.

6. Moteur linéaire selon la revendication 1, 3 ou 4,
**caractérisé en ce qu'**il est prévu un circuit d'évaluation qui génère un signal destiné à indiquer la position du rotor sur la base de la somme des signaux des deux capteurs de champ (15, 16, ou 17, 18).

7. Moteur linéaire selon l'une quelconque des revendications précédentes, **caractérisé en ce qu'**il est prévu un circuit de régulation qui compare une grandeur nominale d'une position du rotor à une valeur de mesure obtenue à partir d'au moins un signal d'au moins un capteur de champ et un signal de réglage destiné à maintenir constante la position du rotor.

8. Moteur linéaire selon l'une quelconque des revendications précédentes, **caractérisé en ce qu'**au moins l'un des capteurs de champ (15, 16) est entouré en direction radiale par l'élément de fermeture arrière (11).

9. Moteur linéaire selon l'une quelconque des revendications précédentes, **caractérisé en ce qu'**au moins une pièce polaire de stator (12, 13) et/ou au moins une pièce polaire de rotor (23, 24, 25) et/ou l'élément de fermeture arrière (11) comprennent au moins un matériau magnétique.

10. Moteur linéaire selon l'une quelconque des revendications précédentes, **caractérisé en ce qu'**un élément optique (51, 52) peut être logé à l'intérieur du rotor (20).

11. Moteur linéaire selon l'une quelconque des revendications précédentes, **caractérisé en ce qu'**un fourreau comportant un matériau non ferromagnétique ayant un faible coefficient de frottement est disposé sur la surface située entre le stator (10) et le rotor (20).

12. Caméra comportant un moteur linéaire selon l'une quelconque des revendications précédentes,
**caractérisé en ce que** le moteur linéaire est prévu pour commander au moins un composant optique.

13. Endoscope comportant un moteur linéaire selon l'une quelconque des revendications précédentes,
**caractérisé en ce que** le moteur linéaire est prévu pour commander au moins un composant optique.

14. Instrument médical selon l'une quelconque des revendications précédentes,
**caractérisé en ce que** le moteur linéaire est prévu pour régler l'angle d'ouverture d'une mâchoire.
